Europäisches Patentamt

European Patent Office

Office européen des brevets

(11) Veröffentlichungsnummer: **0 235 572**
**B1**

(12) **EUROPÄISCHE PATENTSCHRIFT**

(45) Veröffentlichungstag der Patentschrift: **04.04.90**

(51) Int. Cl.⁵: **A 61 N 5/06**

(21) Anmeldenummer: **87101148.2**

(22) Anmeldetag: **28.01.87**

(54) **Bestrahlungsgerät.**

(30) Priorität: **31.01.86 DE 3602965**

(43) Veröffentlichungstag der Anmeldung:
**09.09.87 Patentblatt 87/37**

(45) Bekanntmachung des Hinweises auf die
Patenterteilung:
**04.04.90 Patentblatt 90/14**

(84) Benannte Vertragsstaaten:
**AT BE CH DE ES FR IT LI NL SE**

(56) Entgegenhaltungen:
WO-A-83/02233
DE-A-2 107 929
DE-U-7 613 630
DE-U-7 815 454
FR-A-2 347 943
FR-A-2 445 153

COLOR RESEARCH AND APPLICATION, Band 9,
Nr. 4, Winter 1984, Seiten 195-205, John Wiley &
Sons, Inc., New York, US; P.K. KAISER:
"Phototherapy using chromatic, white, and
ultraviolet light"

(73) Patentinhaber: Weber, Gerhard, Prof. Dr.
Lempenmühle
D-8602 Mühlhausen (DE)

(72) Erfinder: Weber, Gerhard, Prof. Dr.
Lempenmühle
D-8602 Mühlhausen (DE)

(74) Vertreter: Richter, Bernhard, Dipl.-Ing.
Beethovenstrasse 10
D-8500 Nürnberg 20 (DE)

(56) Entgegenhaltungen:

PHYSICS IN MEDECINE & BIOLOGY, Band 23,
Nr. 3, Mai 1978, Seiten 508-510; A.P.
KUSHELEVSKY et al.: "Fibreoptic light guides
for phototesting in dermatological
photobiology"

Courier Press, Leamington Spa, England.

EP 0 235 572 B1

**Beschreibung**

Zur Behandlung des Herpes simplex kennt man, abgesehen von der lokalen äußerlichen Applikation von Antiobiotika, Desoxyuridin und Corticosteroiden (letztere aber verstärken häufig das Wiederauftreten des Herpes simplex), die Photochemotherapie. Bei dieser wird dem Patienten entweder lokal aufgetragen oder systemisch (oral) ein Photosensibilisator, z.B. 8-methoxypsoralen, verabreicht. Danach wird mit einem Bestrahlungsgerät mit Lampen der gesamte Körperteil, in dem der Erkrankungsherd lokalisiert ist, im UVA-Bereich bestrahlt. Dies führt zu eines ungewollten Bestrahlung auch der Umgebung des Krankheitsherdes. Dies aber hat zur Folge, daß die im Herd erzeugte, verstärkte Durchblutung sich auch in nachteiliger Weise auf die gesunde Umgebung erstreckt.

Der Erfindung liegt die Aufgabe der Vermeidung einer als nachteilig empfundenen äußeren oder oralen Verabreichung eines Lichtsensibilisators und ferner der Vermeidung der erläuterten großflächigen Bestrahlung zugrunde.

Zur Lösung dieser Aufgabe ist mit der Erfindung ein Bestrahlungsgerät mit gebündeltem Lichtleiter zur Anwendung für lokale Bestrahlung von Herpes simplex und für die Prophylaxe des rezidivierenden Herpes simplex, mit einer Strahlungsquelle zur Bestrahlung sowohl im UVA-Bereich als auch im UVB-Bereich vorgesehen, mit Mitteln (5) zur Fokussierung der Strahlung auf den krankheitsherd sowie einem Abstandsoder Aufsatzstück, das auf dem den Lichtaustritt aufweisenden Ende des Lichtleiters angebracht und relativ zu dem Ende in der Lichtaustrittsrichtung verstellbar und in der jeweiligen Stellage am Ende fixierbar ist. Hiermit haben sich ganz wesentliche Erfolge beim Herpes simplex und insbesondere auch beim rückfälligen (rezidivierenden) Herpes simplex gezeigt, und zwar ohne eine äußere oder orale Verabreichung eines Lichtsensibilisators. Durch die focussierte, d.h. allein auf den Krankheitsherd beschränkte Bestrahlung mit geündelten UVA-Strahlen (Wellenlänge 315—380 nm) und UVB-Strahlen (Wellenlänge 280—315 nm), oder gemäß Anspruch 2, — diese spektralen Verteilungen können, z.B. durch Verwendung entsprechender Filter erreicht werden — wird eine lokale, nur auf den jeweiligen erkrankten Haut- oder Schleimhautherd beschränkte Hyperämie, also eine Durchblutungssteigerung, erzielt. Bei Applikation einer ausreichenden Strahlendosis kommt es am Ort der Bestrahlung zu einer Provokation des Herpes simplex dergestalt, daß ein existenter, d.h. manifester Herpes simplex exacerbiert, d.h. sich vorübergehend verschlimmert und ein latenter, d.h. zu diesem Zeitpunkt nicht sichtbarer Herpesherd sich manifestiert, d.h. in Erscheinung tritt und zwar unter dem klinischen Vollbild eines Herpes simplex. So ist es möglich, an vorübergehend erscheinungsfreier Haut eines Herpesträgers durch diese Bestrahlung an den Orten vorausgegangener Herpesherde gleichzeitig mehrere isoliert stehende Herpesherde auszulösen und damit die latente, d.h. unsichtbare Infektion mit Herpes simplex-Viren kenntlich zu machen. Das war auf andere Weise medizinisch bisher unmöglich.

Es muß angenommen werden, daß durch die erzeugte Hyperämie körpereigene Abwehrstoffe an den Herpesherd herangeführt werden (z.B. Chalone). Die durch die Bestrahlung erzielte Provokation wird so oft — in Abständen von Tagen — wiederholt bis kein Herpes simplex Herd mehr durch Strahlenprovokation manifestiert, d.h. ausgelöst werden kann. Hierzu genügen in der Regel drei bis sechs Bestrahlungen mit z.B. 0,055 joule/cm². Auf diese Weise ist es gelungen bei Patienten mit Herpes simplex, der in Abständen von 4 Wochen rezidivierte, eine Erscheinungsfreiheit bis zu 5 Jahren zu erzielen, ohne daß zwischenzeitliche Behandlungen notwendig geworden wären. Die geschilderten Nachteile einer ungewollten Bestrahlung der Umgebung des Krankheitsherdes und damit ein Erstrecken der im Herd erzeugten verstärkten Durchblutung auch auf die gesunde Umgebung sind vermieden. Durch das Abstands- oder Aufsatzstück wird zunächst die Focussierung der gebündelten UVA- und UVB-Strahlen nur auf den Krankheitsherd gefördert. Außerdem sichert es einen konstanten Abstand zwischen Strahlenaustrittpunkt und Krankheitsherd. Ohne diese Abstandssicherung würde die Intensität der Bestrahlung von Mal zu Mal entsprechend den jeweiligen Abständen zwischen Krankheitsherd und Strahlenaustrittspunkt variieren. Das hätte sowohl eine unterschiedliche Bestrahlungsqualität auf den Herd als auch eine ungewollte Bestrahlung der gesunden Umgebung des Herdes zur Folge. Das Erythem im bestrahlten Herd hat aber die Aufgabe, körpereigene Abwehrstoffe heranzutransportieren. Würde auch die Herdumgebung mit bestrahlt werden, so würde dies die Abwehrleistung des Organismus im Herdbereich abschwächen.

Zum Stand der Technik wird erwähnt, daß aus FR—A 2 347 943 ein Bestrahlungsgerät zur Anwendung bei Hauterkrankungen, spezielle Psoriasis und Parapsoriasis bekannt ist. Dies aber sind Stoffwechselkrankheiten, während Herpes eine Viruserkrankung ist, so daß insoweit bereits ein prinzipieller Unterschied zur Erfindung besteht. Die zugehörige Apparatur bestrahlt über das gesamte Lichtspektrum von UVC bis zum sehr langwelligen UVA. Das Licht ist nicht gebündelt. Es liegt keine Focussierung vor, da nur an eine Ganzkörperbestrahlung gedacht ist. Auch fehlt das verstellbare Abstandsstück gemäß der Erfindung. FR—A2 445 153 zeigt ein UV-Bestrahlungsgerät zu Zwecken der lokalen Behandlung, das im Impulsbereich von 10 bis 200 Hz arbeitet. Eine Focussierung und ein Abstandsstück fehlen ebenfalls. Aus Physics im Medicine & Biologie, volume 23, No. 3, 1978, Seiten 508—510, ist lediglich ein zylinderförmiges Teil zum Halt eines Filters bekannt, nicht aber ein verstellbares Abstandsoder Aufsatzstück gemäß der Erfindung. Außerdem bezieht sich diese Vorveröffentlichung auch nicht auf ein Gerät nach der Erfindung. Ein Distanzieren der Entfernung zwischen dem Lichtaustritt und der Haut ist nicht möglich. Aus DE—GM 76 13 630 ist weder ein gebündelter Lichtleiter noch ein Gerät und seine Anwendung gemäß der Erfindung vorgesehen. Diese Literaturstelle befaßt sich lediglich mit einem UV-Punktstrahler, wobei eine verschiebbare Aufsatzhülse vorgesehen ist, um den Abstand dieses Punktstrahlers von der Haut zu verändern. Jedoch sind keine Mittel vorgesehen, um den hiermit erreichten Abstand des Punktstrahlers von der Haut zu fixieren und damit für die weitere Anwendung konstant halten zu können.

Die erläuterten Vorteile der Erfindung sind mit keiner dieser Literaturstellen erreichbar.

Die Merkmale des Anspruches 2 beinhalten einen besonders vorteilhaften Wellenlängenbereich.

Zusätzlich ist noch auf die nachstehenden Vorteile der Erfindung hinzuweisen:

— die einfache Handhabung des Bestrahlungsgerätes und damit seiner Anwendung

— der Verzicht auf regelmäßige Behandlungsmaßnahmen bei Rezidiven

— die sichere Überlegenheit gegenüber dem bisher Möglichen

— der geringe Kostenaufwand für die Durchführung der Bestrahlung und

die erfindungsgemäße Möglichkeit zur Rezidivbehandlung, d.h. der Vorbeugung eines an sich bekannten und ruhenden, noch nicht zum Ausbruch gekommenen (latenten) Herpes simplex des Patienten. Der rezidivierende Herpes simplex war bisher therapeutisch unzugänglich und erzeugt beim Patienten oftmals ein echtes Krankheitsbild, ist außerdem kosmetisch störend und psychisch belastend.

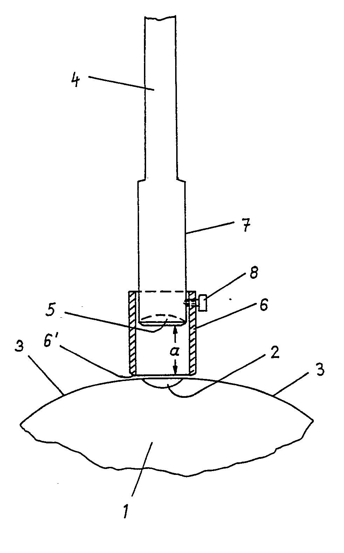

Ein Ausführungsbeispiel der Erfindung ist schematisch in der Zeichnung dargestellt. Am Körperteil 1 ist ein zu bestrahlender Herd 2 vorhanden, dessen Umgebung mit 3 beziffert ist. Über den gebündelten Lichtleiter 4 wird gebündeltes Licht von dem in der Zeichnung nicht dargestellten Bestrahlungsgerät her und mit Hilfe einer Optik 5 oder dergleichen focussiert auf den Herd 2 gestrahlt. Ferner ist ein Abstandsstück 6 vorgesehen, das am Ende 7 des Lichtleiters 4 in dessen Längsrichtung verstellbar und in der gewünschten Stellage fixierbar ist, z.B. mit Hilfe einer angedeuteten Schraube 8. Hiermit kann der Abstand a zwischen Austritt des gebündelten Lichtes aus dem Lichtleiter bzw. der Optik 5 und dem Herd 2 eingestellt und bei der jeweiligen auch bei nachfolgenden Behandlungen konstant gehalten werden. Das Abstands- oder Aufsatzstück 6 gibt auch der die Behandlung durchführenden Person (Krankenschwester) eine gute Handhabe dahingehend, daß sie die Strahlen immer genau auf den Herd 2 bringt. Es verhütet den Austritt der Strahlen nach außen, d.h. in die Umgebung 3, da es mit seiner Mündung 6' fest auf den Körper aufgesetzt werden kann. Die Bestrahlung ist voll auf den Herd 2 konzentriert.

Patentansprüche

1. Bestrahlungsgerät mit gebündeltem Lichtleiter zur Anwendung für lokale Bestrahlung von Herpes simplex und für die Prophylaxe des rezidivierenden Herpes simplex, mit einer Strahlungsquelle zur Bestrahlung sowohl im UVA-Bereich als auch im UVB-Bereich mit Mitteln (5) zur Fokusierung der Strahlung auf den Krankheitsherd sowie einem Abstands- oder Aufsatzstück (6), das auf dem den Lichtaustritt aufweisenden Ende (7) des Lichtleiters (4) angebracht und relativ zu dem Ende (7) in der Lichtaustrittsrichtung verstellbar und in der jeweiligen Stellage am Ende (7) fixierbar (8) ist.

2. Bestrahlungsgerät nach Anspruch 1, mit einer Strahlung im langwelligen Teil des UVB-Bereiches und im kurzweiligen Teil des UVA-Bereiches.

## Revendications

1. Appareil d'irradiation à conducteur optique focalisé desinté à être utilisé pour l'irradiation locale de l'herpès simplex et pour la prophylaxie de l'herpès simplex récidivant, comprenant une source de rayonnement permettant l'irradiation aussi bien dans la gamme de longueurs d'onde des UVA que dans celle des UVB, et comprenant des moyens (5) pour focaliser le rayonnement sur le foyer infectieux, ainsi qu'une pièce d'écartement ou d'application (6) qui est posée sur l'extrémité (7) du conducteur optique (4) présentant la sortie de la lumière, qui peut être déplacée par rapport à l'extrémité (7) dans la direction de la sortie de la lumière et qui peut être fixée (8) à l'extrémité (7) dans chaque position de réglage.

2. Appareil d'irradiation selon la revendication 1, comportant un rayonnement dans la zone des grandes longueurs d'onde de la gamme de longueurs d'onde des UVB et dans la zone des faibles longueurs d'onde de la gamme de longueurs d'onde des UVA.

## Claims

1. Apparatus for applying radiation with a bundled light conductor for the application of local radiation treatment of herpes simplex and for the prophylactic treatment of recidivous herpes simplex, comprising a source of radiation for a radiation treatment in the UVA-range as well as the UVB-range, with means (5) being adapted to focus the radiation towards a focus of the disease, further comprising a distance- or head-piece (6) being attached to the end (7) of the light conductor (4) having the light discharge and which is adjustable relatively to the end (7) in the direction of discharging light and which can be fixed (8) in a respective position on the end (7).

2. Apparatus for applying radiation according to claim 1, comprising a radiation in the longwave part of the UVB-range and in the shortwave part of the UVA-range.